Europäisches Patentamt

(19) European Patent Office

Office européen des brevets

(11) Publication number : **0 416 951 B1**

## (12) EUROPEAN PATENT SPECIFICATION

(45) Date of publication of patent specification :
**12.01.94 Bulletin 94/02**

(51) Int. Cl.⁵ : **A61K 31/57**, // (A61K31/57, 31:135)

(21) Application number : **90309846.5**

(22) Date of filing : **07.09.90**

(54) Medicaments comprising salmeterol and fluticason.

(30) Priority : **08.09.89 GB 8920392**
**20.10.89 GB 8923644**

(43) Date of publication of application :
**13.03.91 Bulletin 91/11**

(45) Publication of the grant of the patent :
**12.01.94 Bulletin 94/02**

(84) Designated Contracting States :
**AT DE DK ES GR LU NL SE**

(56) References cited :
**EP-A- 0 223 671**
**GB-A- 2 107 715**
**GB-A- 2 140 800**
**UNLISTED DRUGS, vol. 33, no. 6, June 1981, Chatnam, NJ (US);pp. 101c**
**MARTINDALE, THE EXTRA PHARMACOPOEIA, 29th ed., 1989; p. 882**

(73) Proprietor : **GLAXO GROUP LIMITED**
**Clarges House 6-12 Clarges Street**
**London W1Y 8DH (GB)**

(72) Inventor : **Palmer, James Barry Douglas**
**Glaxo Group Research Limited, Berkeley Avenue**
**Greenford, Middlesex UB6 0HE (GB)**

(74) Representative : **Marsden, John Christopher et al**
**Frank B. Dehn & Co. Imperial House 15-19 Kingsway**
**London WC2B 6UZ (GB)**

## Description

This invention relates to improvements in the treatment of asthma and other respiratory disorders. More particularly, it relates to the use of a bronchodilator drug in combination with a steroidal anti-inflammatory drug for the treatment of respiratory disorders such as asthma, and to pharmaceutical compositions containing the two active ingredients.

Asthma is a condition characterised by variable, reversible obstruction of the airways which is caused by a complex inflammatory process within the lungs. In most cases, this process is initiated and maintained by the inhalation of antigens by sensitive atopic individuals (extrinsic asthma). However, in some patients it is caused by other mechanisms which at present are poorly understood but do not involve an allergic process (intrinsic asthma). The disease has therefore two components, spasm of the bronchial (or breathing) tubes and inflammation or swelling of the breathing tubes.

Salbutamol, the first highly selective $\beta_2$-adrenoceptor stimulant has been used successfully and effectively by inhalation for the immediate relief of spasm in asthma. However, when given by inhalation, salbutamol has usually a four to six hour duration of action, which is too short either to control nocturnal asthma or for convenient maintenance of the disease in some patients.

Anti-inflammatory corticosteroids such as, for example, beclomethasone dipropionate have also been administered by inhalation in the treatment of asthma, although unlike salbutamol the therapeutic benefits resulting from reduced inflammation may not be immediately apparent.

It has been recognised that asthma may be treated by using both a bronchodilator for immediate relief and a prophylactic anti-inflammatory corticosteroid to treat the underlying inflammation. Such combination therapy directed at the two main underlying events in the lung (i.e. relief of spasm in the breathing tubes and treatment of inflammation in the breathing tubes) using a combination of salbutamol and beclomethasone dipropionate has previously been proposed (Ventide, Glaxo Group trade mark), but suffers a number of disadvantages in view of the above-mentioned short duration of action exhibited by salbutamol. Thus the need for a 4-hourly dosing regimen may discourage effective patient compliance and also renders the product less than satisfactory in the treatment of nocturnal asthma since the bronchodilator may not remain effective for the duration of the night, leading to impaired sleep for asthmatics troubled by nocturnal cough, breathlessness and wheeze.

The present invention is based on the concept of a novel combination therapy which has markedly greater efficiency and duration of bronchodilator action than previously known combinations and which permits the establishment of a twice daily (bis in diem - b.i.d) dosing regimen with consequent substantial benefits in, for example, the treatment of asthma, particularly nocturnal asthma.

Thus we have found that if the $\beta_2$-adrenoreceptor stimulant brochodilator salmeterol and/or a physiologically acceptable salt thereof is combined with the anti-inflammatory corticosteroid fluticasone propionate in a form suitable for administration by inhalation, the resulting compositions may be administered on a b.i.d. basis to provide highly effective treatment and/or prophylactic therapy for asthmatics. In particular such administration has been shown to lead to significant improvement in daytime lung function, requirement for additional symptomatic bronchodilator and almost complete abolition of nocturnal asthma while giving rise to minimal systemic side effects.

Salmeterol is one of a range of bronchodilators having extended duration of action which is described in GB-A- 2140800, and is systematically named 4-hydroxy-$\alpha^1$-[[[6-(4-phenylbutoxy)hexyl]amino]methyl]-1,3-benzenedimethanol. Fluticasone propionate is one of a range of topical anti-inflammatory corticosteroids with minimal liability to undesired systemic side effects which is described in GB-A- 2088877, and is systematically named S-fluoromethyl 6$\alpha$,9$\alpha$-difluoro-11$\beta$-hydroxy-16$\alpha$-methyl-17$\alpha$-propionyloxy-3-oxoandrosta-1,4- diene-17$\beta$-carbothioate. We have found these two compounds to be particularly compatible and complementary in their activity and thus highly effective in the treatment of asthma and other respiratory disorders.

Thus according to one aspect of the invention there are provided pharmaceutical compositions comprising effective amounts of salmeterol and/or a physiologically acceptable salt thereof and fluticasone propionate as a combined preparation for simultaneous administration by inhalation in the treatment of respiratory disorders.

The invention additionally relates to the use of salmeterol and/or a physiologically acceptable salt thereof and fluticasone propionate in the manufacture of pharmaceutical compositions as combined preparations for simultaneous administration of salmeterol and fluticasone propionate by inhalation in the treatment of respiratory disorders.

Suitable physiologically acceptable salts of salmeterol include acid addition salts derived from inorganic and organic acids, such as the hydrochloride, hydrobromide, sulphate, phosphate, maleate, tartrate, citrate, benzoate, 4-methoxybenzoate, 2- or 4-hydroxybenzoate, 4-chlorobenzoate, p-toluenesulphonate, methanesulphonate, ascorbate, salicylate, acetate, fumarate, succinate, lactate, glutarate, gluconate, tricarballylate, hy- droxynaphthalenecarboxylate e.g. 1-hydroxy- or 3-hydroxy-2-naphthalenecarboxylate, or oleate. Salmeterol is preferably used in the form of its 1-hydroxy-2-naphthalene carboxylate salt (hydroxynaphthoate).

For administration by inhalation, the compositions according to the invention are conveniently delivered by conventional means, e.g. in the form of a metered dose inhaler prepared in a conventional manner or in combination with a spacer device such as the Volumatic (Glaxo Group trade mark) device. In the case of a metered dose inhaler, a metering valve is provided to deliver a metered amount of the composition. Spray compositions may for example be formulated as aqueous solutions or suspensions and may be administered by a nebuliser. Aerosol spray formulations, for example in which the active ingredients are suspended, optionally together with one or more stabilisers, in a propellant, e.g. a halogenated hydrocarbon such as trichlorofluoromethane, dichlorodifluoromethane, 1,2-dichlorotetrafluoroethane, trichlorotrifluoroethane, monochloropentafluoroethane, chloroform or methylene chloride, may also be employed.

Alternatively, for administration by inhalation or insufflation, the compositions according to the invention may take the form of a dry powder composition, for example a powder mix of the active ingredients and a suitable carrier such as lactose. The powder compositions may be presented in unit dosage form in, for example, capsules, cartridges or blister packs from which the powder may be administered with the aid of an inhaler such as the Rotahaler inhaler (Glaxo Group trade mark) or in the case of blister packs by means of the Diskhaler inhaler (Glaxo Group trade mark).

The ratio of salmeterol to fluticasone propionate in the compositions according to the invention is preferably within the range 4:1 to 1:20. Each metered dose or actuation of the inhaler will generally contain from 25 µg to 100 µg of salmeterol and from 25 µg to 500 µg of fluticasone propionate. As hereinbefore indicated, it is intended that the pharmaceutical compositions will be administered twice daily.

A suitable daily dose of salmeterol for inhalation is in the range 50 µg to 200 µg.

A suitable daily dose of fluticasone propionate for inhalation is in the range 50 µg to 2000 µg depending on the severity of the disease.

The precise dose employed will of course depend on the method of administration, the age, weight and condition of the patient and will be determined by the clinician depending on the severity and the type of asthma.

In order that the invention may be more fully understood, the following examples are given.

EXAMPLE 1 - Metered Dose Inhaler

| Active Ingredient | Target per Actuation | Per Inhaler % w/w |
|---|---|---|
| Salmeterol (as hydroxynaphthoate) | 25.0 µg | 0.0448 |
| Fluticasone propionate | 25.0 µg | 0.0309 |
| Stabiliser | 5.0 µg | 0.0076 |
| Trichlorofluoromethane | 23.70 mg | 27.8759 |
| Dichlorodifluoromethane | 61.25 mg | 72.0588 |

EXAMPLE 2 - Metered Dose Inhaler

| Active Ingredient | Target per Actuation | Per Inhaler % w/w |
|---|---|---|
| Salmeterol (as hydroxynaphthoate) | 25.0 μg | 0.0448 |
| Fluticasone propionate | 50.0 μg | 0.0618 |
| Stabiliser | 7.5 μg | 0.0106 |
| Trichlorofluoromethane | 23.67 mg | 27.8240 |
| Dichlorodifluoromethane | 61.25 mg | 72.0588 |

EXAMPLE 3 - Metered Dose Inhaler

| Active Ingredient | Target per Actuation | Per Inhaler % w/w |
|---|---|---|
| Salmeterol (as hydroxynaphthoate) | 25.0 μg | 0.0448 |
| Fluticasone propionate | 250.0 μg | 0.3088 |
| Stabiliser | 25.0 μg | 0.0309 |
| Trichlorofluoromethane | 23.45 mg | 27.5567 |
| Dichlorodifluoromethane | 61.25 mg | 72.0588 |

4

EXAMPLE 4 - Metered Dose Inhaler

| Active Ingredient | Target per Actuation | Per Inhaler % w/w |
|---|---|---|
| Salmeterol (as hydroxynaphthoate) | 25.0 µg | 0.0448 |
| Fluticasone propionate | 125.0 µg | 0.1544 |
| Stabiliser | 15.0 µg | 0.0175 |
| Trichlorofluoromethane | 23.56 mg | 27.7244 |
| Dichlorodifluoromethane | 61.25 mg | 72.0588 |

EXAMPLE 5 - Metered Dose Inhaler

| Active Ingredient | Target per Actuation | Per Inhaler % w/w |
|---|---|---|
| Salmeterol (as hydroxynaphthoate) | 100.0 µg | 0.1791 |
| Fluticasone propionate | 250.0 µg | 0.3088 |
| Stabiliser | 25.0 µg | 0.0309 |
| Trichlorofluoromethane | 23.43mg | 27.4224 |
| Dichlorodifluoromethane | 61.25 mg | 72.0588 |

In Examples 1 to 5 micronised fluticasone propionate and micronised salmeterol (as the hydroxynaphthoate) are added in the proportions given above either dry or after predispersal in a small quantity of stabiliser (disodium dioctylsulphosuccinate, lecithin, oleic acid or sorbitan trioleate)/trichlorofluoromethane solution to a suspension vessel containing the main bulk of the trichlorofluoromethane solution. The resulting suspension is further dispersed by an appropriate mixing system using, for example, a high shear blender, ultrasonics or a microfluidiser until an ultrafine dispersion is created. The suspension is then continuously recirculated to suitable filling equipment designed for cold fill or pressure filling of dichlorodifluoromethane. Alternatively, the suspension may be prepared in a suitable chilled solution of stabiliser, in trichlorofluoromethane/dichlorodifluoromethane.

5

### EXAMPLE 6 - Metered Dose Dry Powder Formulation

| Active Ingredient | | µg/cartridge or blister |
|---|---|---|
| Salmeterol (as hydroxynaphthoate) | | 36.3 |
| Fluticasone propionate | | 50.00 |
| Lactose Ph.Eur. | to | 12.5 mg or |
| | to | 25.0mg |

### EXAMPLE 7 - Metered Dose Dry Powder Formulation

| Active Ingredient | | µg/cartridge or blister |
|---|---|---|
| Salmeterol (as hydroxynaphthoate) | | 72.5 |
| Fluticasone propionate | | 50.00 |
| Lactose Ph.Eur. | to | 12.5 mg or |
| | to | 25.0 mg |

### EXAMPLE 8 - Metered Dose Dry Powder Formulation

| Active Ingredient | | µg/cartridge or blister |
|---|---|---|
| Salmeterol (as hydroxynaphthoate) | | 72.5 |
| Fluticasone propionate | | 100.00 |
| Lactose Ph.Eur. | to | 12.5 mg or |
| | to | 25.0 mg |

### EXAMPLE 9 - Metered Dose Dry Powder Formulation

| Active Ingredient | | ug/cartridge or blister |
|---|---|---|
| Salmeterol (as hydroxynaphthoate) | | 72.5 |
| Fluticasone propionate | | 250 |
| Lactose Ph.Eur. | to | 12.5 mg or |
| | to | 25.0 mg |

### EXAMPLE 10 - Metered Dose Dry Powder Formulation

| Active Ingredient | | µg/cartridge or blister |
|---|---|---|
| Salmeterol (as hydroxynaphthoate) | | 72.5 |
| Fluticasone propionate | | 500.0 |
| Lactose Ph. Eur. | to | 12.5 mg or |
| | to | 25.0 mg |

### EXAMPLE 11 - Metered Dose Dry Powder Formulation

| Active Ingredient | | µg/cartridge or blister |
|---|---|---|
| Salmeterol (as hydroxynaphthoate) | | 145.0 |
| Fluticasone propionate | | 250.0 |
| Lactose Ph. Eur. | to | 12.5 mg or |
| | to | 25.0 mg |

In Examples 6 to 11 the active ingredients are micronised and bulk blended with the lactose in the proportions given above. The blend is filled into hard gelatin capsules or cartridges or in specifically constructed double foil blister packs (Rotadisks blister packs, Glaxo Group trade mark) to be administered by an inhaler such as the Rotahaler inhaler (Glaxo Group trade mark) or in the case of the blister packs with the Diskhaler inhaler (Glaxo Group trade mark).

## Claims

### Claims for the following Contracting States : AT, DK, DE, LU, NL, SE

1. Compositions containing salmeterol and/or a physiologically acceptable salt thereof and fluticasone propionate for simultaneous administration by inhalation in the treatment of respiratory disorders.

2. Compositions as claimed in claim 1 wherein salmeterol is present as its 1-hydroxy-2-naphthoate salt.

3. Compositions as claimed in claim 1 or claim 2 presented in the form of a metered dose inhaler or a metered dry powder composition.

4. Compositions as claimed in any of claims 1 to 3 in dosage unit form containing 25-100µg of salmeterol optionally in the form of a physiologically acceptable salt thereof and 25-500µg of fluticasone propionate per dosage unit.

5. The use of salmeterol and/or a physiologically acceptable salt thereof and fluticasone propionate in the manufacture of pharmaceutical compositions for simultaneous administration of salmeterol and fluticasone propionate by inhalation in the treatment of respiratory disorders.

6. The use of salmeterol and/or a physiologically acceptable salt thereof and fluticasone propionate according to claim 5 in the manufacture of pharmaceutical compositions for administration on a twice daily basis.

### Claims for the following Contracting States : GR, ES

1. The use of salmeterol and/or a physiologically acceptable salt thereof and fluticasone propionate in the manufacture of pharmaceutical compositions for simultaneous administration of salmeterol and fluticasone propionate by inhalation in the treatment of respiratory disorders.

2. The use according to claim 1 wherein salmeterol is present as its 1-hydroxy-2-naphthoate salt.

3. The use of salmeterol and/or a physiologically acceptable salt thereof and fluticasone propionate according to claim 1 or claim 2 in the manufacture of compositions presented as a metered spray composition or a dry powder composition.

4. The use of salmeterol and/or a physiologically acceptable salt thereof and fluticasone propionate according to any of claims 1 to 3 in the manufacture of compositions in dosage unit form containing 25-100µg of salmeterol optionally in the form of a physiologically acceptable salt thereof and 25-500µg of fluticasone propionate per dosage unit.

5. The use of salmeterol and/or a physiologically acceptable salt thereof and fluticasone propionate according to any of claims 1 to 4 in the manufacture of pharmaceutical compositions for administration on a twice daily basis.

## Patentansprüche

### Patentansprüche für folgende Verstragsstaaten : AT, DK, DE, LU, NL, SE

1. Mittel, enthaltend Salmeterol und/oder ein physiologisch verträgliches Salz davon und Fluticasonpropionat zur gleichzeitigen Verabreichung durch Inhalieren bei der Behandlung respiratorischer Erkrankungen.

2. Mittel nach Anspruch 1, wobei Salmeterol als sein 1-Hydroxy-2-naphthoatsalz vorliegt.

**3.** Mittel nach Anspruch 1 oder 2, vorliegend in einem abgemessene Mengen dosierenden Inhalator oder in Form eines vordosierten trockenen Pulvermittels.

**4.** Mittel nach einem der Ansprüche 1 bis 3 in Ein heitsdosierform, enthaltend 25 bis 100 μg Salmeterol, gegebenenfalls in Form eines physiologisch verträglichen Salzes davon, und 25 bis 500 μg Fluticasonpropionat pro Dosiereinheit.

**5.** Verwendung von Salmeterol und/oder einem physiologisch verträglichen Salz davon und Fluticasonpropionat bei der Herstellung von Arzneimitteln zur gleichzeitigen Verabreichung von Salmeterol und Fluticasonpropionat durch Inhalieren bei der Behandlung respiratorischer Erkrankungen.

**6.** Verwendung von Salmeterol und/oder einem physiologisch vertraglichen Salz davon und Fluticasonpropionat nach Anspruch 5 bei der Herstellung von Arzneimitteln zur Verabreichung auf einer zweimal taglichen Basis.

**Patentansprüche für folgende Vertragsstaaten : GR, ES**

**1.** Verwendung von Salmeterol und/oder einem physiologisch verträglichen Salz davon und Fluticasonpropionat bei der Herstellung von Arzneimitteln zur gleichzeitigen Verabreichung von Salmeterol und Fluticasonpropionat durch Inhalieren bei der Behandlung respiratorischer Erkrankungen.

**2.** Verwendung nach Anspruch 1, wobei Salmeterol als sein 1-Hydroxy-2-naphthoatsalz vorliegt.

**3.** Verwendung von Salmeterol und/oder einem physiologisch verträglichen Salz davon und Fluticasonpropionat nach Anspruch 1 oder 2 bei der Herstellung von Mitteln, vorliegend als eine abgemessene Sprühzusammensetzung oder ein trockenes Pulvermittel .

**4.** Verwendung von Salmeterol und/oder einem physiologisch verträglichen Salz davon und Fluticasonpropionat nach einem der Ansprüche 1 bis 3 bei der Herstellung von Mitteln in Einheitsdosierform, enthaltend 25 bis 100 μg Salmeterol, gegebenenfalls in Form eines physiologisch verträglichen Salzes davon, und 25 bis 500 μg Fluticasonpropionat pro Dosiereinheit.

**5.** Verwendung von Salmeterol und/oder einem physiologisch verträglichen Salz davon und Fluticasonpropionat nach einem der Ansprüche 1 bis 4 bei der Herstellung von Arzneimitteln zur Verabreichung auf einer zweimal täglichen Basis.

**Revendications**

**Revendications pour les Etats contractants suivants : AT, DK, DE, LU, NL, SE**

**1.** Compositions contenant du salmétérol et/ou un sel physiologiquement acceptable de celui-ci et du propionate de fluticasone pour l'administration simultanée par inhalation dans le traitement des affections respiratoires.

**2.** Compositions selon la revendication 1, dans lesquelles le salmétérol est présent sous forme de son sel 1-hydroxy-2-naphtoate.

**3.** Compositions selon la revendication 1 ou 2, présentées sous la forme de doses bien déterminées pour inhalation ou de compositions dosées de poudre sèche.

**4.** Compositions selon l'une quelconque des revendications 1 à 3, sous forme de doses unitaires contenant de 25 à 100 μg de salmétérol, facultativement sous la forme d'un sel physiologiquement acceptable de celui-ci et 25 à 500 μg de propionate de fluticasone par dose unitaire.

**5.** Utilisation du salmétérol et/ou d'un sel physiologiquement acceptable de celui-ci et du propionate de fluticasone pour la préparation de compositions pharmaceutiques pour l'administration simultanée de salmétérol et de propionate de fluticasone par inhalation dans le traitement des affections respiratoires.

**6.** Utilisation du salmétérol et/ou d'un sel physiologiquement acceptable de celui-ci et du propionate de fluticasone selon la revendication 5, pour la préparation de compositions pharmaceutiques en vue de l'administration biquotidienne.

**Revendications pour les Etats contractants suivants : GR, ES**

1.  Utilisation du salmétérol et/ou d'un sel physiologiquement acceptable de celui-ci et du propionate de fluticasone pour la préparation de compositions pharmaceutiques pour l'administration simultanée de salmétérol et de propionate de fluticasone par inhalation dans le traitement des affections respiratoires.

2.  Utilisation selon la revendication 1, dans laquelle le salmétérol est présent sous forme de son sel 1-hydroxy-2-naphtoate.

3.  Utilisation du salmétérol et/ou d'un sel physiologiquement acceptable de celui-ci et du propionate de fluticasone selon la revendication 1 ou 2, pour la préparation de compositions présentées sous forme de compositions dosées à pulvériser ou de compositions en poudre sèche.

4.  Utilisation du salmétérol et/ou d'un sel physiologiquement acceptable de celui-ci et de propionate de fluticasone selon l'une quelconque des revendications 1 à 3, pour la préparation de compositions sous forme de doses unitaires contenant 25 à 100 μg de salmétérol facultativement sous la forme d'un sel physiologiquement acceptable de celui-ci et 25 à 500 μg de propionate de fluticasone par dose unitaire.

5.  Utilisation du salmétérol et/ou d'un sel physiologiquement acceptable de celui-ci et du propionate de fluticasone selon l'une quelconque des revendications 1 à 4 pour la préparation de compositions pharmaceutiques en vue de l'administration biquotidienne.